# EUROPEAN PATENT APPLICATION

(11) **EP 3 144 692 A2**
(43) Date of publication of application: **22.03.2017**
(21) Application number: 16156272.3
(22) Date of filing: 18.02.2016
(51) Int. Cl.: G01R 33/567, G01R 33/54

(54) **MAGNETIC RESONANCE IMAGING WITH ADJUSTMENT OF IMAGING PARAMETERS BASED ON RESPIRATORY DATA**

(30) Priority: 10.09.2015 KR 20150128328
(71) Applicant: Samsung Electronics Co., Ltd., Suwon-si, Gyeonggi-do (KR)
(72) Inventor: Hwang, Jin Young, Gyeonggi-do (KR); Jung, Sung Pil, Gyeonggi-do (KR)
(74) Representative: Gulde & Partner

(57) **Abstract**

A magnetic resonance imaging apparatus and a method of acquiring a magnetic resonance image are provided. The magnetic resonance imaging apparatus includes a data acquirer configured to acquire image data of a subject based on a protocol, the protocol including a learning protocol for learning a respiration of the subject and determining an initial protocol based on the learned respiration. The magnetic resonance imaging apparatus further includes a respiratory information acquirer configured to detect the respiration of the subject, and a protocol controller configured to determine whether a respiratory cycle of the subject changes based on the detected respiration, and in response to the protocol controller determining that the respiratory cycle of the subject changes, reset the protocol based on the changed respiratory cycle.

## Description

### BACKGROUND

### 1. Field

Apparatuses and methods consistent with exemplary embodiments relate to a magnetic resonance imaging apparatus for diagnosing various diseases using magnetic resonance images and a method of acquiring the magnetic resonance images.

### 2. Description of the Related Art

In general, medical imaging apparatuses acquire information on a patient and provide an image. Medical imaging apparatuses include ultrasonic diagnostic apparatuses, X-ray computed tomography apparatuses, magnetic resonance imaging apparatuses, medical diagnostic apparatuses, etc. Among these apparatuses, magnetic resonance imaging apparatuses play an important role in the medical diagnostic field using medical images because magnetic resonance imaging apparatuses have relatively free image capture conditions, and provide excellent contrast in soft tissues and various diagnostic information images.

Magnetic resonance imaging apparatuses are for diagnosing the interior of a human body by applying a predetermined frequency and energy to atomic nuclei under the influence of a magnetic field, and converting energy emitted from the atomic nuclei due to nuclear magnetic resonance into signals.

In detail, magnetic resonance imaging apparatuses may represent a ratio of the intensity of a magnetic resonance (MR) signal to the intensity of a radio frequency (RF) signal generated in an intensity of a magnetic field through contrast, and may acquire a tomography image of the subject.

Recently, research has been conducted on several methods for acquiring a clear magnetic resonance image without affecting the state of the subject.

### SUMMARY

Exemplary embodiments address at least the above problems and/or disadvantages and other disadvantages not described above. Also, the exemplary embodiments are not required to overcome the disadvantages described above, and may not overcome any of the problems described above.

One or more exemplary embodiments provide a magnetic resonance imaging apparatus for acquiring a clear magnetic resonance image irrespective of the respiration of the subject, and a method of acquiring the magnetic resonance image.

According to an aspect of an exemplary embodiment, there is provided a magnetic resonance imaging apparatus including a data acquirer configured to acquire image data of a subject based on a protocol, the protocol including a learning protocol for learning a respiration of the subject and determining an initial protocol based on the learned respiration. The magnetic resonance imaging apparatus further includes a respiratory information acquirer configured to detect the respiration of the subject, and a protocol controller configured to determine whether a respiratory cycle of the subject changes based on the detected respiration, and in response to the protocol controller determining that the respiratory cycle of the subject changes, reset the protocol based on the changed respiratory cycle.

The protocol controller may be further configured to reset an acquisition time of the image data based on the changed respiratory cycle.

The protocol controller may be further configured to reset a number of slices of the image data that are acquired during a cycle of the respiration based on the changed respiratory cycle.

The protocol controller may be further configured to increase the number of slices in response to the protocol controller determining that the respiratory cycle lengthens, and decrease the number of slices in response to the protocol controller determining that the respiratory cycle shortens.

The protocol controller may be further configured to reset the protocol such that a slice that is not acquired within the decreased number of slices is reacquired.

The protocol controller may be further configured to reset the protocol such that a slice that is acquired immediately before the respiratory cycle changes is reacquired.

The protocol controller may be further configured to reset a slice acquisition time of a slice of the image data based on the changed respiratory cycle.

The protocol controller may be further configured to increase the slice acquisition time in response to the protocol controller determining that the respiratory cycle lengthens, and decrease the slice acquisition time in response to the protocol controller determining that the respiratory cycle shortens.

The protocol controller may be further configured to set the protocol such that the detection of the respiration of the subject and the acquisition of the image data are alternately performed.

The protocol controller may be further configured to reset the protocol such that the image data is acquired during an expiration of the changed respiratory cycle.

The protocol may include an image acquisition protocol for the acquisition of the image data, and the image acquisition protocol may include a scanning time period for the acquisition of the image data, a navigator time period for acquiring navigator data for the detection of the respiration of the subject, and an idle time period to be provided between the scanning time period and the navigator time period.

According to an aspect of another exemplary embodiment, there is provided a method of acquiring a magnetic resonance image, the method including acquiring image data of a subject based on a protocol, the protocol including a learning protocol for learning a respiration of the subject and determining an initial protocol based on the learned respiration. The method further includes detecting the respiration of the subject, determining whether a respiratory cycle of the subject changes based on the detected respiration, and in response to the determining that the respiratory cycle of the subject changes, resetting the protocol based on the changed respiratory cycle.

The resetting may include resetting an acquisition time of the image data based on the changed respiratory cycle.

The resetting may include resetting a number of slices of the image data that are acquired during a cycle of the respiration based on the changed respiratory cycle.

The resetting may include increasing the number of slices in response to the determining that the respiratory cycle lengthens, and decreasing the number of slices in response to the determining that the respiratory cycle shortens.

The method may further include resetting the protocol such that a slice that is not acquired within the decreased number of slices is reacquired.

The resetting may include resetting a slice acquisition time of a slice of the image data based on the changed respiratory cycle.

The resetting may include increasing the slice acquisition time in response to the determining that the respiratory cycle lengthens, and decreasing the slice acquisition time in response to the determining that the respiratory cycle shortens.

The method may further include setting the protocol such that the detecting of the respiration of the subject and the acquiring of the image data are alternately performed.

The detecting may include detecting the respiration of the subject based on a change in a position of a thoracic diaphragm.

According to an aspect of another exemplary embodiment, there is provided a magnetic resonance imaging apparatus including a data acquirer configured to acquire image data of a subject based on a protocol, the protocol including a time period for the acquisition of the image data during an expiration of a respiration of the subject. The magnetic resonance imaging apparatus further includes a respiratory information acquirer configured to detect the respiration, and a protocol controller configured to determine whether a respiratory cycle of the subject changes based on the detected respiration, and in response to the protocol controller determining that the respiratory cycle changes, reset the time period based on the changed respiratory cycle.

The protocol controller may be further configured to increase a number of slices of the image data that are acquired during a cycle of the respiration in response to the protocol controller determining that the respiratory cycle lengthens, and decrease the number of slices in response to the protocol controller determining that the respiratory cycle shortens

The protocol controller may be further configured to increase a slice time period for an acquisition of a slice of the image data in response to the protocol controller determining that the respiratory cycle lengthens, and decrease the slice time period in response to the protocol controller determining that the respiratory cycle shortens.

### BRIEF DESCRIPTION OF THE DRAWINGS

The above and/or other aspects will be more apparent by describing exemplary embodiments with reference to the accompanying drawings, in which:
FIG. 1 is a block diagram of a magnetic resonance imaging apparatus, according to an exemplary embodiment;
FIG. 2 is a diagram schematically showing a thoracic diaphragm;
FIG. 3 is a diagram showing a change in a thoracic diaphragm according to respiration of a subject;
FIG. 4 is a diagram for describing acquisition of MR data and creation of a magnetic resonance image;
FIG. 5 is a diagram for describing a protocol for acquiring a magnetic resonance image, according to an exemplary embodiment;
FIG. 6 is a diagram for describing in detail an image acquisition protocol, according to an exemplary embodiment;
FIGS. 7A and 7B are diagrams showing an error in acquiring MR data according to a change in a respiratory cycle of a subject, according to an exemplary embodiment;
FIG. 8 is a diagram showing a change of a protocol caused by a change in a respiratory cycle of a subject, according to an exemplary embodiment;
FIG. 9 is a diagram showing a change of a protocol when a respiratory cycle of a subject lengthens, according to an exemplary embodiment;
FIG. 10 is a diagram showing a change of a protocol when a respiratory cycle of a subject lengthens, according to another exemplary embodiment;
FIG. 11 is a diagram showing a change of a protocol when a respiratory cycle of a subject shortens, according to another exemplary embodiment;
FIG. 12 is a diagram showing a change of a protocol when a respiratory cycle of a subject shortens, according to another exemplary embodiment;
FIG. 13 is a diagram showing a change of a protocol when a respiratory cycle of a subject shortens, according to still another exemplary embodiment;
FIG. 14 is a diagram for describing reacquisition of image data according to a change of a respiratory cycle, according to another exemplary embodiment;
FIG. 15 is a perspective view schematically showing an exterior of a magnetic resonance imaging system, according to an exemplary embodiment;
FIG. 16 is a control block diagram for describing a magnetic resonance imaging system, according to an exemplary embodiment;
FIG. 17 is a cutout view for describing a magnet assembly, according to an exemplary embodiment;
FIG. 18 is a diagram showing structures of a magnet assembly and a gradient coil, according to an exemplary embodiment;
FIG. 19 is a flowchart showing a method of acquiring a magnetic resonance image in a magnetic resonance imaging system, according to an exemplary embodiment; and
FIG. 20 is a flowchart for describing in detail the method of acquiring a magnetic resonance image of FIG. 19.

### DETAILED DESCRIPTION

Exemplary embodiments are described in greater detail below with reference to the accompanying drawings.

In the following description, like drawing reference numerals are used for like elements, even in different drawings. The matters defined in the description, such as detailed construction and elements, are provided to assist in a comprehensive understanding of the exemplary embodiments. However, it is apparent that the exemplary embodiments can be practiced without those specifically defined matters. Also, well-known functions or constructions may not be described in detail because they would obscure the description with unnecessary detail.

It will be understood that the terms "comprises" and/or "comprising" used herein specify the presence of stated features or components, but do not preclude the presence or addition of one or more other features or components. In addition, the terms such as "unit", "-er (-or)", and "module" described in the specification refer to an element for performing at least one function or operation, and may be implemented in hardware, software, or the combination of hardware and software.

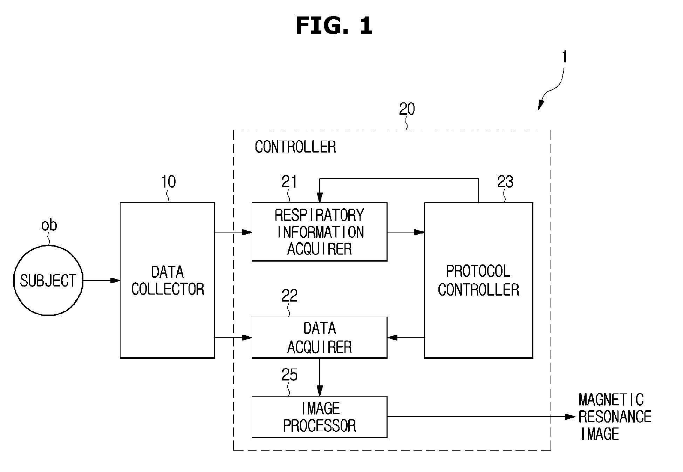

FIG. 1 is a block diagram of a magnetic resonance imaging apparatus 1, according to an exemplary embodiment.

Referring to FIG. 1, the magnetic resonance imaging apparatus 1 includes a data collector 10 and a controller 20. The magnetic resonance imaging apparatus 1 is an apparatus for exposing a subject (ob) to a magnetic field and then acquiring an image on the subject (ob) through a magnetic resonance phenomenon. An image obtained through the magnetic resonance phenomenon is a magnetic resonance image.

A representative example of the subject (ob) may be a human body, but is not limited thereto. An animal may be the subject (ob).

The data collector 10 may collect magnetic resonance data. The magnetic resonance data (hereinafter referred to as MR data) may include a magnetic resonance signal created by nuclear magnetic resonance (NMR), in which nuclei constituting the subject (ob) resonate with predetermined electromagnetic waves.

In addition, the data collector 10 may preprocess the MR data received from the subject (ob). In detail, the data collector 10 may amplify the MR data or convert an analog signal into an electric signal and output the electric signal.

The MR data includes navigator data and image data. Here, the navigator data denotes MR data used to track respiration of the subject (ob), and the image data denotes MR data used to create a magnetic resonance image of the subject (ob).

The data collector 10 may alternately collect the navigator data and the image data according to a protocol. The protocol used to collect the navigator data and the image data will be described in detail below.

The controller 20 controls the overall operation of the magnetic resonance imaging apparatus 1. The controller 20 may correspond to a single processor or a plurality of processors. The processor may be implemented by an array of logic gates, or may be implemented in a combination of a general-purpose microprocessor and a memory in which programs to be executed by the microprocessor are stored. For example, the processor may be implemented as a general-purpose device such as a central processing unit (CPU) or graphics processing unit (GPU), but is not limited thereto.

In addition, the controller 20 may output a control signal to each component constituting the magnetic resonance imaging apparatus 1, acquire image data, that is, MR data regarding the subject (ob), and create a magnetic resonance image on the basis of the acquired image data.

The image data may be acquired according to a predetermined protocol. The controller 20 controls a magnetic field or high frequency waves that are applied to the subject (ob) to acquire the image data according to the predetermined protocol.

In addition, the controller 20 may set a protocol according to respiration of the subject (ob). To prevent noise (artifacts) caused by the respiration of the subject (ob), the protocol may be set according to the respiration of the subject (ob).

That is, the controller 20 may set a protocol such that the image data is acquired during expiration in which the subject (ob) exhales internal air to the outside, thus minimizing the noise caused by the respiration of the subject (ob).

In addition, the controller 20 may track a change in respiration of the subject (ob) and dynamically reset a protocol according to the change in respiration. In this way, the controller 20 may dynamically change the protocol according to the change in respiration of the subject (ob), thus minimizing the noise caused by the respiration and minimizing an acquisition time of the magnetic resonance image.

To this end, the controller 20 includes a respiratory information acquirer 21, a data acquirer 22, image processor 25, and a protocol controller 23.

The respiratory information acquirer 21 tracks respiration of the subject (ob). In addition, the respiratory information acquirer 21 may acquire respiratory information by tracking the respiration of the subject (ob).

Here, the respiratory information includes a variety of information that may be derived from the respiration of the subject (ob). For example, the respiratory information acquirer 21 may acquire a respiratory cycle by tracking the respiration of the subject (ob). As another example, the respiratory information acquirer 21 may acquire a respiratory gradient of the subject (ob) by tracking the respiration of the subject (ob).

Here, the respiratory gradient is inversely proportional to the respiratory cycle of the subject (ob). For example, a short respiratory cycle of the subject (ob) denotes fast respiration of the subject (ob). In other words, the short respiratory cycle denotes heavy respiration of the subject (ob). Accordingly, as the respiratory cycle shortens, the respiratory gradient increases. On the other hand, a long respiratory cycle of the subject (ob) denotes slow respiration of the subject (ob). Accordingly, as the respiratory cycle lengthens, the respiratory gradient decreases.

Additionally, the respiratory information acquirer 21 may detect the respiration of the subject (ob) and may acquire respiratory information using various parameters representing a respiratory state of the subject (ob), but is not limited thereto.

The respiratory information acquirer 21 may acquire the respiratory information through various devices for detecting the respiration of the subject (ob).

For example, the respiratory information acquirer 21 may include a respiratory navigator that detects the respiration of the subject (ob). As another example, the respiratory information acquirer 21 may include an air tube or a belt, etc. According to an exemplary embodiment, the respiratory information acquirer 21 may insert an air tube between a torso coil and an abdominal area of the subject (ob) and may acquire respiratory information on the basis of a change in air pressure in the air tube. Additionally, the respiratory information acquirer 21 may acquire respiratory information through various well-known methods, but is not limited thereto.

As an example of respiration detection methods, a respiration detection method of the respiratory information acquirer 21 including the respiratory navigator will be described below. However, exemplary embodiments are not limited thereto.

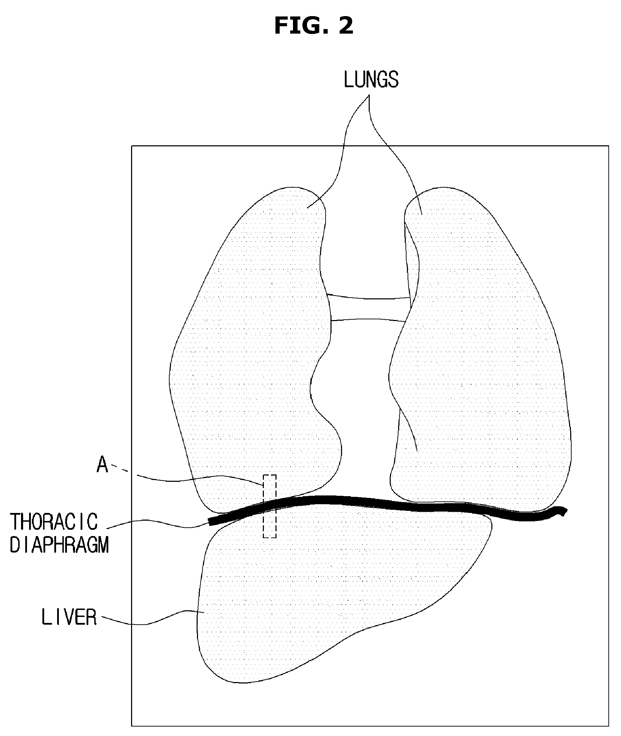

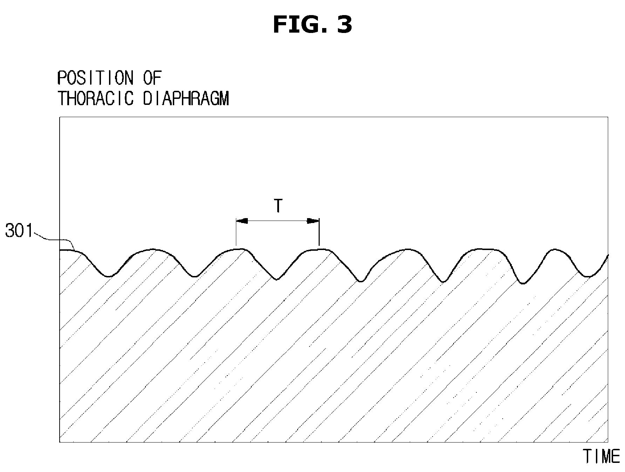

FIG. 2 is a diagram schematically showing a thoracic diaphragm. FIG. 3 is a diagram showing a change in the thoracic diaphragm according to respiration of a subject (ob). In FIG. 3, a horizontal axis indicates time, and a vertical axis indicates a location or position of the thoracic diaphragm.

Referring to FIGS. 2 and 3, the respiratory information acquirer 21 may track the respiration of the subject (ob) on the basis of MR data collected by the data collector 10.

A location of the thoracic diaphragm is changed according to the respiration of the subject (ob). The thoracic diaphragm is a sheet that separates the thoracic cavity from the abdominal cavity. As shown in FIG. 2, the lungs and the liver are separated by the thoracic diaphragm.

The thoracic diaphragm moves up toward the lungs during expiration in which air is exhaled by the subject (ob) and moves down toward the liver during inspiration in which air is inhaled by the subject (ob). That is, the thoracic diaphragm moves up and down according to the respiration of the subject (ob).

Thus, the respiratory information acquirer 21 may track the respiration of the subject (ob) using movement of the thoracic diaphragm according to the subject (ob). The respiratory information acquirer 21 may track the movement of the thoracic diaphragm from navigator data transmitted to the data collector 10. In this case, the navigator data is MR data acquired at a corresponding position of the thoracic diaphragm. The respiratory information acquirer 21 may track a motion of the thoracic diaphragm from a navigator image acquired by performing image processing on the navigator data.

The respiratory information acquirer 21 may directly track the movement of the thoracic diaphragm in the navigator image, but may also track the movement of the thoracic diaphragm using anatomical information.

For example, because the positions of the lungs and liver adjacent to the thoracic diaphragm change according to the movement of the thoracic diaphragm, the respiratory information acquirer 21 may track the movement of the thoracic diaphragm according to the movement of a boundary line formed by the lungs and the liver.

In detail, a histogram such as that in FIG. 3 may be acquired by displaying navigator images on a region A of FIG. 2 in chronological order. A boundary line 301 included in the histogram is positioned between the lungs and the liver and corresponds to the movement of the thoracic diaphragm.

That is, because the lungs expand and the liver contracts during inspiration, the boundary line 301 between the lungs and the liver moves down. Accordingly, the movement of the boundary line 301 down indicates inspiration.

On the other hand, because the lungs contract and the liver expands during expiration, the boundary line 301 between the lungs and the liver moves up. Accordingly, the movement of the boundary line 301 up indicates expiration.

For example, respiratory information of the subject (ob) acquired from the respiratory information acquirer 21 may be transferred to the protocol controller 23 and then used to set a protocol for acquiring MR data.

The respiratory information acquirer 21 has been described as tracking the respiration of the subject (ob) on the basis of the navigator image. However, the method of tracking the respiration of the subject (ob) is not limited thereto. For example, the respiration of the subject (ob) may be tracked by a predetermined sensor attached to the subject (ob).

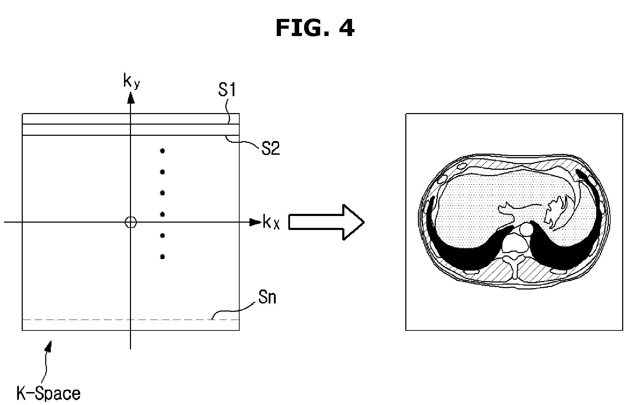

FIG. 4 is a diagram for describing acquisition of MR data and creation of magnetic resonance images.

Referring to FIGS. 1 and 4, the data acquirer 22 may acquire image data regarding a k-space according to a protocol set by the protocol controller 23.

The k-space denotes a set of raw data for creating one magnetic resonance image. The data acquirer 22 fills raw data in the k-space with the image data acquired according to the protocol.

To acquire a clear magnetic resonance image as described above, image data is acquired during expiration. Thus, the data acquirer 22 may sequentially acquire image data during expiration according to the protocol and fill raw data of one k-space with the acquired image data.

The data acquirer 22 may acquire image data for each slice and may fill the k-space through the acquisition. That is, as shown in FIG. 4, the data acquirer 22 may acquire the image data in units of slices S1, S2, ..., Sn. Because each of the slices S1, S2, ..., Sn with which the k-space is filled has a different position through phase and frequency encoding, a phase-encoding gradient magnetic field and a frequency-encoding gradient magnetic field may be applied differently for each slice.

An image data acquisition timing for each individual slice S1, S2, ..., Sn may be determined according to a protocol determined by the protocol controller 23 that will be described below. When the k-space is filled with the slices S1, S2, ..., Sn, the data acquirer 22 transfers the image data regarding the k-space to the image processor 25.

The image processor 25 creates the magnetic resonance image on the basis of the image data regarding the k-space transmitted from the data acquirer 22. In detail, as shown in FIG. 4, the image processor 25 may perform the Fourier transform on the image data regarding the k-space to create a magnetic resonance image.

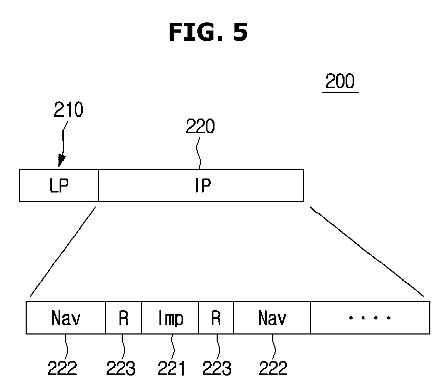

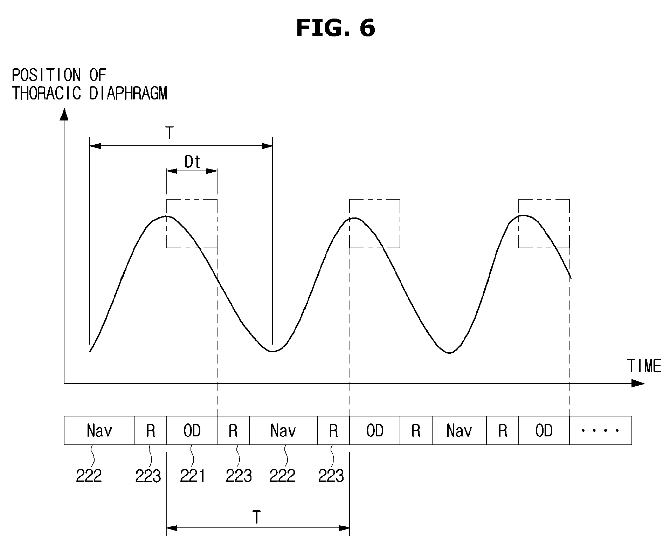

FIG. 5 is a diagram for describing a protocol 200 for acquiring a magnetic resonance image, according to an exemplary embodiment. FIG. 6 is a diagram for describing in detail an image acquisition protocol 220, according to an exemplary embodiment.

Referring to FIGS. 1 and 5, the protocol controller 23 may set the protocol 200 for acquiring a magnetic resonance image and may transmit a control signal to the respiratory information acquirer 21 and the data acquirer 22 according to the set protocol 200.

As shown in FIG. 5, the protocol 200 includes a learning protocol 210 (LP) for learning the respiration of the subject (ob) and an image acquisition protocol 220 (IP) for acquiring image data.

In the learning protocol 210, respiratory information of the subject (ob) is learned. To acquire a clear magnetic resonance image as described above, image data is acquired during expiration. Accordingly, to set the image acquisition protocol 220, only the respiration of the subject (ob) is learned in the learning protocol 210 without acquisition of the image data of the subject (ob), and thus the image acquisition protocol 220 is set.

That is, in the learning protocol 210, the respiratory information acquirer 21 continuously tracks the respiration of the subject (ob). In this case, the respiration of the subject (ob) may be tracked during a predetermined time, but is not limited thereto. For example, in the learning protocol 210, the learning of the subject (ob) may continue until the respiratory cycle or respiratory gradient of the subject (ob) stabilizes or until the accuracy reaches a predetermined threshold value.

When the learning of the respiration of the subject (ob) ends, the protocol controller 23 may set the image acquisition protocol 220 on the basis of the respiration of the subject (ob) that is learned by a learning processor.

The image acquisition protocol 220 includes a scanning period 221 (Imp or OD) configured to acquire image data of the subject (ob) and a navigator period 222 (Nav) configured to acquire navigator data for detecting the respiration of the subject (ob).

The protocol controller 23 may alternately arrange the scanning period 221 and the navigator period 222 to create the image acquisition protocol 220. In this case, an idle period 223 (R) is provided between the scanning period 221 and the navigator period 222 to remove a signal regarding a section of no interest.

Through the idle period 223, equilibrium (rest) magnetization may be performed on the signal regarding the section of no interest, and thus aliasing may be avoided.

The protocol controller 23 may set the scanning period 221 of the image acquisition protocol 220 on the basis of the respiratory information of the subject (ob) acquired by the learning protocol 210.

In detail, as shown in FIG. 6, the protocol controller 23 may set the image acquisition protocol 220 such that the scanning period 221 is performed during expiration according to the respiratory information of the subject (ob). The scanning period 221 may be set at an interval corresponding to a respiratory cycle T and may acquire the image data during expiration.

Here, the length of the scanning period 221 may be determined according to the respiratory information of the subject (ob).

For example, the length of the scanning period 221 may be determined according to the respiratory cycle T. As the respiratory cycle T of the subject (ob) lengthens, a duration Dt of expiration increases. As the respiratory cycle T of the subject (ob) shortens, the duration Dt of expiration decreases.

That is, because the respiratory cycle T and expiratory duration Dt of the subject (ob) are proportional to each other, the protocol controller 23 may determine the length of the scanning period 221 on the basis of the respiratory cycle T.

In addition, the length of the scanning period 221 may be determined according to the respiratory gradient of the subject (ob). As the respiratory gradient of the subject (ob) decreases, the expiratory duration Dt increases. As the respiratory gradient of the subject (ob) increases, the expiratory duration Dt decreases.

That is, because the expiratory duration Dt is proportional to the respiratory cycle T of the subject (ob) or inversely proportional to the respiratory gradient of the subject (ob), the protocol controller 23 may determine the length of the scanning period on the basis of the respiratory cycle T or the respiratory gradient.

As shown in FIG. 6, the protocol controller 23 may set a region corresponding to expiration as the navigator period 222 according to the respiratory information of the subject (ob). The protocol controller 23 may set a data acquisition time for the subject (ob) and an acquisition time of the navigator data for tracking the respiration of the subject (ob) to be different from each other by setting the navigator period 222 not to overlap the scanning period 221.

In this way, by setting the navigator period 222 between scanning periods 221, the protocol controller 23 may track the respiration of the subject (ob) during an image data acquisition period of the subject (ob).

The protocol controller 23 may set the idle period 223 between the navigator period 222 and the scanning period 221, thus avoiding aliasing of the MR data.

As a result, the image acquisition protocol 220 may be set such that the navigator period 222, the idle period 223, the scanning period 221, and the idle period 223 are repeatedly performed, and the lengths and periods of the navigator period 222, the idle period 223, and the scanning period 221 may be determined according to at least one of the respiratory cycle and the respiratory gradient.

In this way, it is possible to calculate an optimal image data acquisition timing by learning the respiration of the subject (ob) first and then setting the image acquisition protocol 220 according to the learned respiratory cycle of the subject (ob).

In addition, it is also possible to minimize an image data acquisition time by adjusting an image data acquisition time (the length of the scanning period 221) in advance according to the respiration of the subject (ob).

Furthermore, it is also possible to acquire a clear magnetic resonance image by adjusting an image data acquisition timing and an acquisition time according to the learned respiratory cycle.

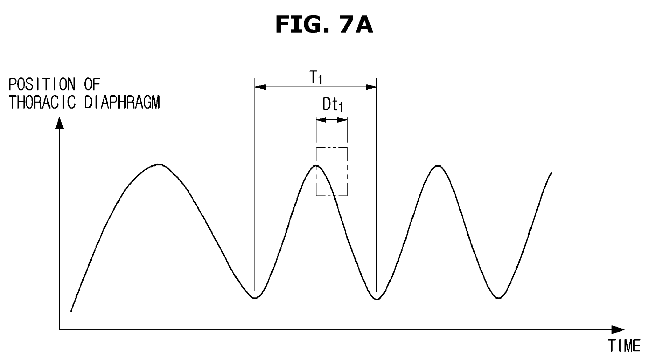

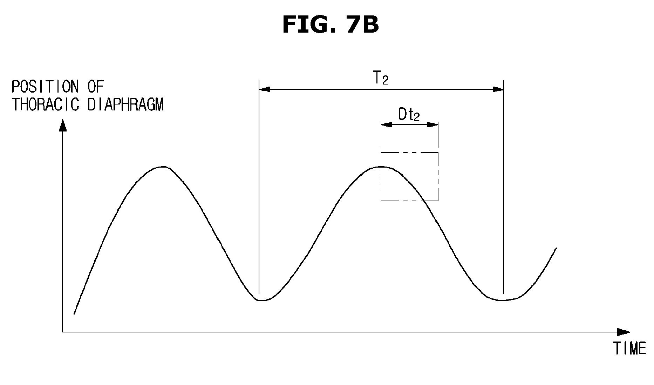

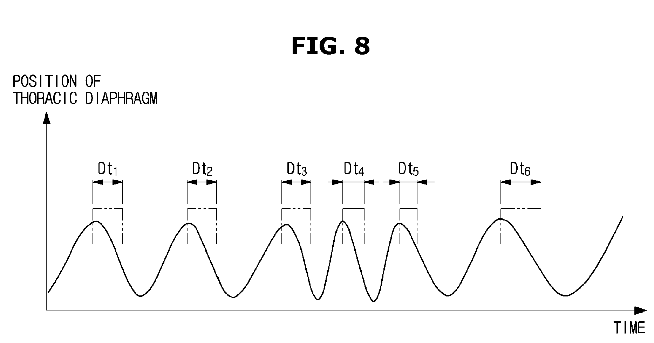

FIGS. 7A and 7B are diagrams showing an error in acquiring MR data according to a change in a respiratory cycle of a subject (ob), according to an exemplary embodiment. FIG. 8 is a diagram showing a change of a protocol caused by a change in a respiratory cycle of a subject (ob), according to an exemplary embodiment.

As described above, the image acquisition protocol 220 alternately has the navigator period 222 and the scanning period 221. That is, the data collector 10 alternately collects navigator data and image data.

The data acquirer 22 fills raw data of k-space using the image data acquired by the data collector 10.

The respiratory information acquirer 21 uses the navigator data acquired in the navigator period 222 to track respiration of the subject (ob) during capturing the magnetic resonance image, and delivers the tracked respiration to the protocol controller 23.

However, the image data of the subject (ob) cannot be efficiently acquired using the image acquisition protocol 220 in which the respiration of the subject (ob) is set according to the respiratory information acquired through the learning while the magnetic resonance image is captured.

In detail, referring to FIGS. 6 and 7A, when the respiratory cycle of the subject (ob) changes from T to T1, the length of expiration decreases from Dt to Dt1, and a start time of expiration changes. On the other hand, referring to FIGS. 6 and 7B, when the respiratory cycle of the subject (ob) changes from T to T2, the length of expiration increases from Dt to Dt2, and the start time of expiration changes.

Accordingly, when the image data is acquired by applying the image acquisition protocol 220 created based on the respiratory cycle T, the quality of the magnetic resonance image may be degraded due to noise caused by the respiration of the subject (ob).

Furthermore, referring to FIGS. 6 and 7A, when the respiratory gradient of the subject (ob) changes from L to L1, the length of expiration decreases from Dt to Dt1, and a start time of expiration changes. On the other hand, referring to FIGS. 6 and 7B, when the respiratory gradient of the subject (ob) changes from L to L2, the length of expiration increases from Dt to Dt2, and the start time of expiration changes.

Thus, the protocol controller 23 may determine whether the respiration of the subject (ob) has changed on the basis of the respiration of the subject (ob) acquired while the magnetic resonance image is captured. When the respiration of the subject (ob) has changed, the protocol controller 23 may change the set image acquisition protocol 220 on the basis of the changed respiration.

A method of adjusting the length of an image data acquisition time according to a change in a respiratory cycle will be described below. However, because the respiratory cycle and the respiratory gradient are inversely proportional to each other as described above, the length of the image data acquisition time may also be adjusted according to a change in the respiratory gradient.

For example, as shown in FIG. 8, the protocol controller 23 may change the length Dt1 to Dt6 of the scanning period 221 of the image acquisition protocol 220 according to the change in the respiratory cycle of the subject (ob), and may adjust the length of the image data acquisition time of the subject (ob).

As described above, as the respiratory cycle of the subject (ob) shortens, the length of expiration also decreases relatively. Accordingly, when the respiratory cycle shortens, the protocol controller 23 may decrease the length of the scanning period 221 and then decrease a time taken to acquire the image data of the subject (ob), thus preventing the quality of the magnetic resonance image from being degraded.

On the other hand, when the respiratory cycle of the subject (ob) lengthens, the length of expiration also increases. Accordingly, when the respiratory cycle lengthens, the protocol controller 23 may increase the length of the scanning period 221 for which the image data is acquired, increase a time taken to acquire the image data of the subject (ob), and decrease a capture time of the magnetic resonance image.

That is, as shown in FIG. 8, the protocol controller 23 may dynamically reset the length Dt1 to Dt6 of the scanning period 221 according to the change in the respiratory cycle of the subject (ob), and thus may dynamically change the length of the image data acquisition time.

In this way, when the length of the scanning period 221 is changed according to the change in the respiratory cycle, a sequence of the scanning period 221 may be changed. That is, the protocol controller 23 may change the number of slices acquired in one scanning period 221 and the size of an acquisition window according to the length of the scanning period 221. The change of the sequence of the scanning period 221 will be described in detail below.

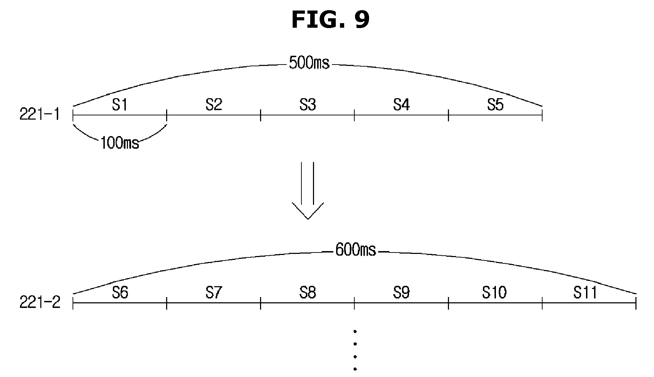

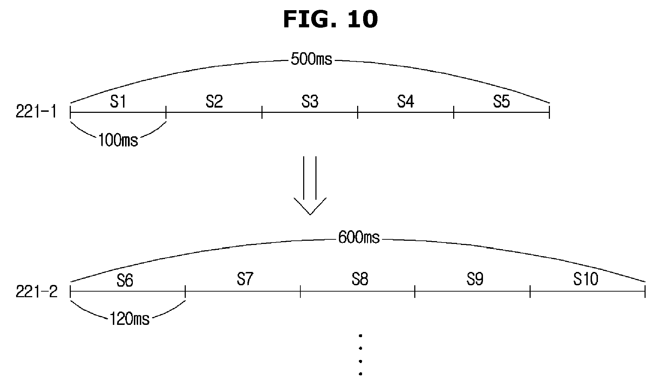

FIG. 9 is a diagram showing a change of the protocol 200 when a respiratory cycle of a subject (ob) lengthens, according to an exemplary embodiment. FIG. 10 is a diagram showing a change of the protocol 200 when a respiratory cycle of a subject (ob) lengthens, according to another exemplary embodiment. FIGS. 9 and 10 are diagrams showing a change of a sequence when the respiratory cycle of the subject (ob) lengthens as shown in FIG. 7B.

As shown in FIG. 7B, when the respiratory cycle of the subject (ob) lengthens from T to T2, the length of expiration increases from Dt to Dt2. The length of the scanning period 221 is changed according to a respiratory change, and thus an acquisition time of image data in one respiratory cycle increases from Dt to Dt2. The protocol controller 23 may change the sequence of the scanning period 221 on the basis of the increased image data acquisition time.

Referring to FIGS. 9 and 10, five slices S1 to S5 may be acquired in a scanning period 221-1 before the respiratory cycle lengthens. That is, the data acquirer 22 acquires one slice in units of 100 ms.

When the respiratory cycle of the subject (ob) lengthens while the image data is acquired, the protocol controller 23 increases the length of a scanning period 221-2 to be subsequently executed from 500 ms to 600 ms in response to the change in the respiratory cycle. Thus, a time taken to acquire image data during one breath increases from 500 ms to 600 ms.

In this way, when the length of the scanning period 221-2 increases, the protocol controller 23 may change a sequence of the scanning period 221-2.

As an example, the protocol controller 23 may increase the number of slices acquired during one scanning period 221-2. Here, the increased number of slices may be determined according to the increased length of the scanning period 221.

For example, as shown in FIG. 9, when the length of the scanning period 221 increases by 100 ms, the protocol controller 23 may change the number of slices acquired during one scanning period from five to six. Thus, in the scanning period 221-2 after the respiratory cycle lengthens, six slices S6 to S11 are acquired during one breath.

In this way, it is possible to reduce a time taken to acquire the magnetic resonance image of the subject (ob) by increasing the number of slices acquired during one cycle of the respiration.

As another example, the protocol controller 23 may change the size of the slice acquisition window according to lengthening of the respiratory cycle. Here, the size of the slice acquisition window may be determined according to the increased length of the scanning period 221.

For example, as shown in FIG. 10, when the length of the scanning period 221 increases by 100 ms, the protocol controller 23 may increase the size of the data acquisition window used to acquire each slice from 100 ms to 120 ms, thus further enhancing an accuracy of the slice.

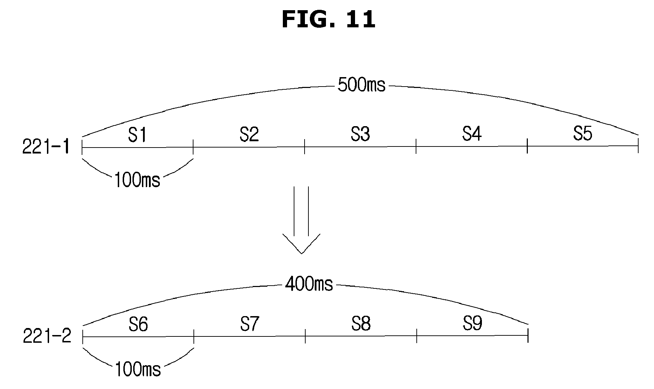

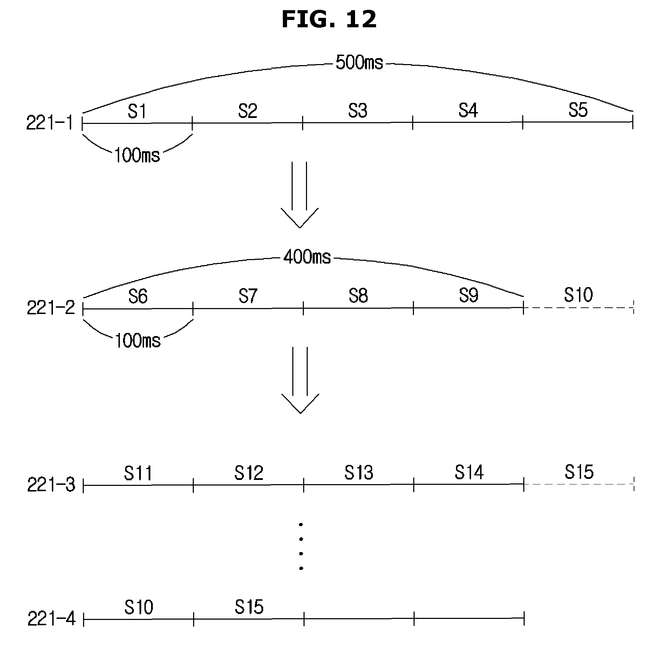

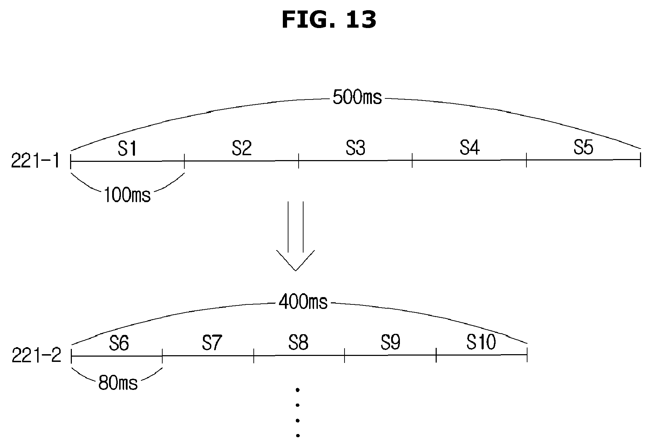

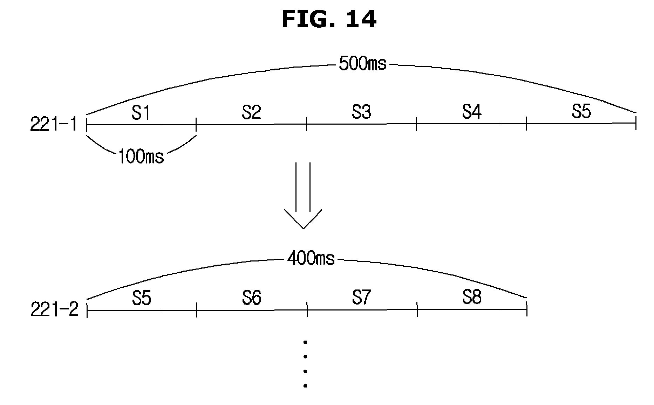

FIG. 11 is a diagram showing a change of a protocol when a respiratory cycle of a subject (ob) shortens, according to an exemplary embodiment. FIG. 12 is a diagram showing a change of a protocol when a respiratory cycle of a subject (ob) shortens, according to another exemplary embodiment. FIG. 13 is a diagram showing a change of a protocol when a respiratory cycle of a subject (ob) shortens, according to still another exemplary embodiment. FIG. 14 is a diagram for describing reacquisition of image data according to a change of a respiratory cycle, according to an exemplary embodiment.

As shown in FIG. 7A, when the respiratory cycle of the subject (ob) shortens from T to T1, the length of expiration decreases from Dt to Dt1. The length of the scanning period 221 is shortened according to such a respiratory change. Because the length of the scanning period 221 is changed according to the respiratory change, an image data acquisition time during one cycle of the respiration decreases from Dt to Dt1. The protocol controller 23 may change a sequence of the scanning period 221 on the basis of the decreased image data acquisition time.

Referring to FIGS. 11 to 13, five slices S1 to S5 may be acquired in the scanning period 221 before the respiratory cycle shortens. That is, the data acquirer 22 acquires image data of one slice in units of 100 ms.

When the respiratory cycle of the subject (ob) shortens while the image data is acquired, the protocol controller 23 decreases the length of the scanning period 221-2 to be subsequently executed from 500 ms to 400 ms in response to the change in the respiratory cycle. Thus, a time taken to acquire image data during one breath decreases from 500 ms to 400 ms.

In this way, as the length of the scanning period 221 decreases, the protocol controller 23 may change the sequence of the scanning period 221, thus preventing the loss of the acquired image data.

As an example, the protocol controller 23 may decrease the number of slices acquired during one scanning period 221. Here, the decreased number of slices may be determined according to the decreased length of the scanning period 221.

For example, as shown in FIG. 11, when the length of the scanning period 221 decreases by 100 ms, the protocol controller 23 may change the number of slices acquired during one scanning period 221 from five to four. Thus, in the scanning period 221-2 after the respiration changes, four slices S5 to S9 or S10 to S13 are acquired during one breath.

In this way, it is possible to prevent deterioration in quality of the magnetic resonance image by decreasing the number of slices acquired in one scanning period 221 according to the respiratory cycle.

In FIG. 11, it has been described that slices are sequentially acquired. However, a slice acquisition order is not limited thereto.

For example, as shown in FIG. 12, the slices may be acquired according to the predetermined protocol 200, but slices S10 and S15 that are not acquired because the scanning period 221 is shortened may be acquired through a separate scanning period 221-k. That is, the protocol controller 23 may additionally create a scanning period 221 for acquiring the slices that are not acquired because the scanning period 221-k is shortened.

As another example, the protocol controller 23 may change the size of the slice acquisition window according to shortening of the respiratory cycle. Here, the size of the slice acquisition window may be determined according to the decreased length of the scanning period 221.

For example, as shown in FIG. 13, when the length of the scanning period 221 decreases by 100 ms, the protocol controller 23 may decrease the size of the data acquisition window used to acquire each slice from 100 ms to 80 ms.

In this way, by decreasing the size of the data acquisition window, the protocol controller 23 may secure a magnetic resonance image capturing speed, irrespective of the respiration of the subject (ob).

When the respiratory cycle shortens, the protocol controller 23 may change the scanning period 221 to reacquire some or all of the image data acquired before the respiratory cycle shortens.

As described above, the respiration of the subject (ob) is tracked through the navigator period 222 that is executed alternately with the scanning period 221. Accordingly, when the respiration of the subject (ob) changes rapidly while the scanning period 221 is in progress, some of the image data is not acquired during expiration and thus the loss of the image data may occur.

Thus, when the respiratory cycle of the subject (ob) shortens, the protocol controller 23 may set the scanning period 221 such that the image data acquired in a previous scanning period 221 is reacquired. Here, the reacquisition of the image data may be performed in units of slices.

Referring to FIG. 14, for example, when the respiratory change of the subject (ob) is sensed, the length of a second scanning period 221-2 is changed according to the respiratory change of the subject (ob), and thus the number of slices acquired in the scanning period 221 may decrease.

When the respiration of the subject (ob) previously changes in the first scanning period 221-1 before the respiratory change is sensed by the protocol controller 23, some slices acquired in the first scanning period 221-1 may not be acquired during expiration. Thus, the protocol controller 23 may set the second scanning period 221-2 such that any slice S5 before the respiratory change is sensed is reacquired.

In this way, the protocol controller 23 may reacquire image data that has been acquired before the respiratory cycle of the subject (ob) shortens, thus preventing deterioration in quality of the magnetic resonance image due to the rapid respiratory change of the subject (ob).

A magnetic resonance imaging system including the magnetic resonance imaging apparatus 1 will be described in detail below.

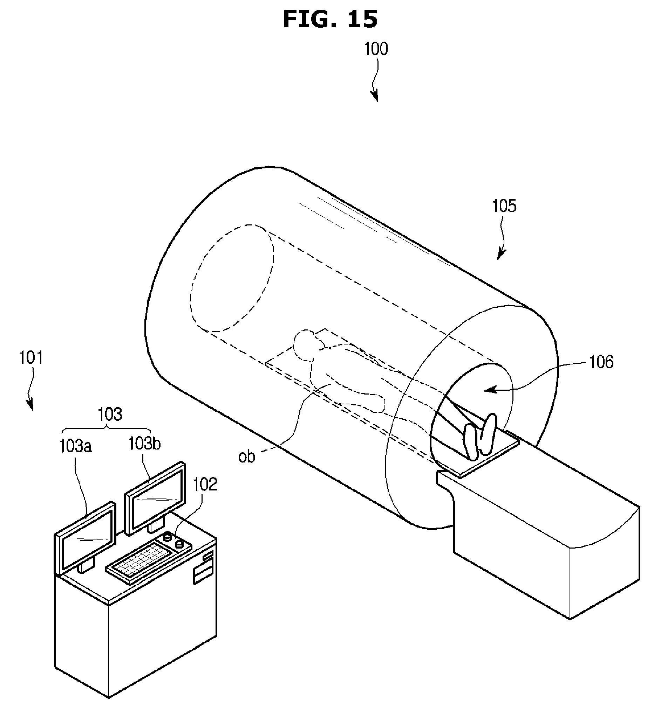

FIG. 15 is a perspective view schematically showing an exterior of a magnetic resonance imaging system 100, according to an exemplary embodiment.

Referring to FIG. 15, the magnetic resonance imaging system 100 includes an operating console 101 for a user controlling the magnetic resonance imaging system 100 and a main body 105 for generating a magnetic field and generating a resonance phenomenon for atomic nuclei. Here, the "user" may be a medical expert including a doctor, a nurse, a medical laboratory scientist, a medical imaging expert, or a technician that repairs a medical device, but is not limited thereto.

The operating console 101 and the main body 105 may be provided in different spaces. In detail, the operating console 101 may be provided in an operating room, and the main body 105 may be provided in a scan room.

The operating console 101 includes an operating interface 102 for receiving a control command from the user and a display 103 for providing information to the user.

The operating interface 102 receives the control command from the user and outputs an electric signal corresponding to the input control command. The user may use the operating interface 102 to enter or select information regarding the subject (ob), parameter information, a scan condition, a pulse sequence, and information regarding image composition or differential operations. In addition, the user may use the operating interface 102 to set a k-space in which a magnetic resonance image will be acquired or to set a protocol.

The operating interface 102 may be implemented with a button input interface such as a keyboard, a push button, or a membrane button or a touch input interface such as a touch pad, but is not limited thereto. For example, the operating interface 102 may be implemented with a trackball, a voice recognition device, or a gesture recognition device or may be implemented within a range known to those skilled in the art.

The display 103 may output a magnetic resonance image or output a variety of information used by the user to control the magnetic resonance imaging system 100. In addition, the display 103 includes a plurality of displays 103a and 103b, which may display different magnetic resonance images from each other. One display 103a may display a magnetic resonance image, and the other display 103b may output a variety of information used to control the magnetic resonance imaging system 100.

The main body 105 may be a gantry having a hollow cylinder. In this case, an inner space of the cylinder is called a bore 106 or a cavity. A magnet assembly 150 for generating a magnetic field and generating a resonance phenomenon for atomic nuclei may be provided in the main body 105. A configuration of the magnetic resonance imaging system 100 will be described below in detail.

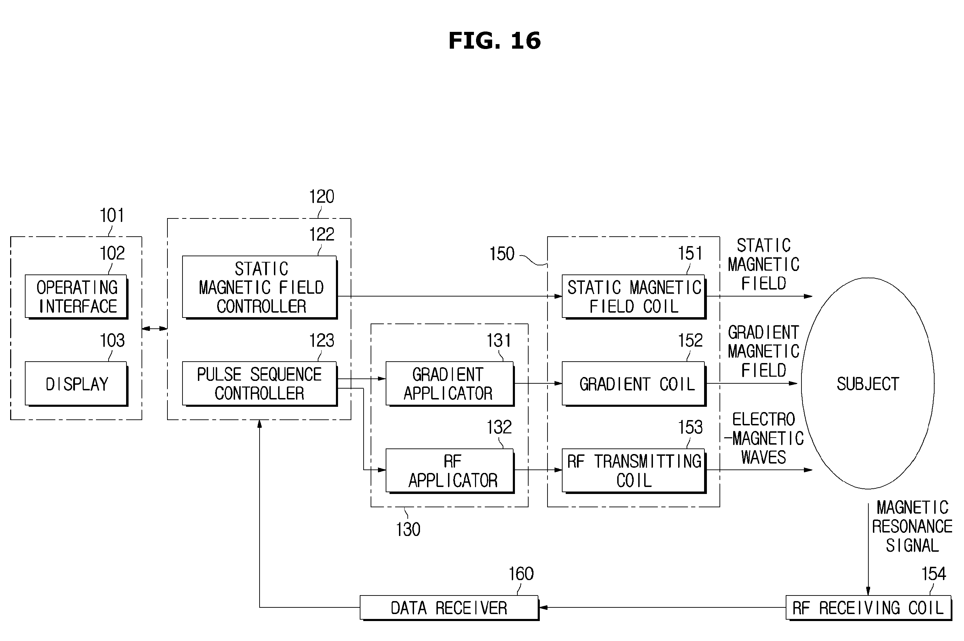

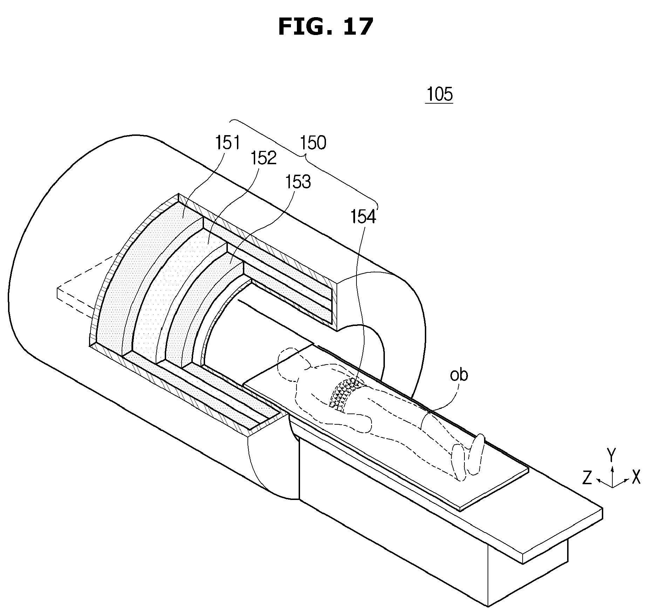

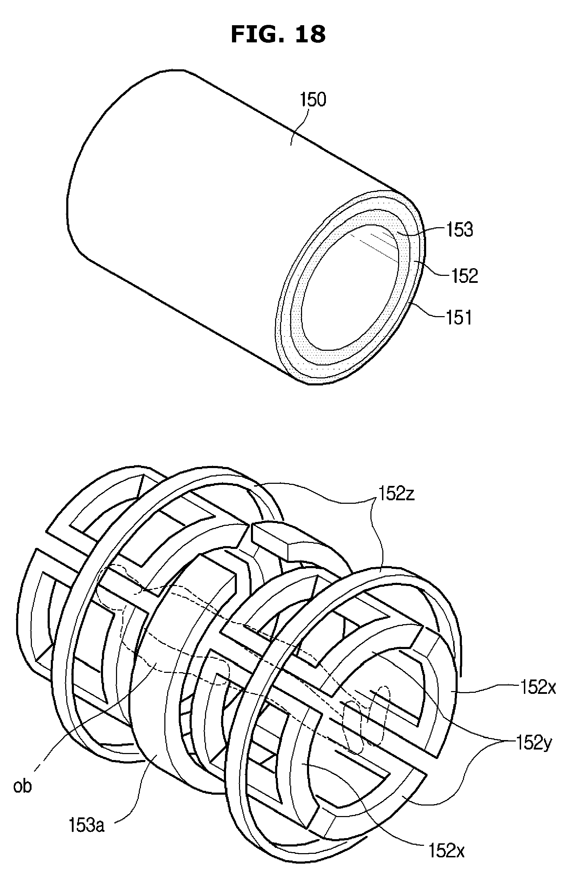

FIG. 16 is a control block diagram for describing the magnetic resonance imaging system 100, according to an exemplary embodiment. FIG. 17 is a cutout view for describing a magnet assembly 150, according to an exemplary embodiment. FIG. 18 is a diagram showing structures of the magnet assembly 150 and a gradient coil 152, according to an exemplary embodiment.

Referring to FIGS. 16 and 18, the magnetic resonance imaging system 100 includes the magnetic assembly 150 for generating a magnetic field and generating a resonance phenomenon for atomic nuclei, an applicator 130 for applying an electrical current to the magnetic assembly 150 to generate the magnetic field and the resonance phenomenon, a data receiver 160 for receiving echo signals generated from the atomic nuclei, that is, magnetic resonance data, the operating console 101 for monitoring a state of the apparatus, and a main controller 120 for controlling the overall operation of the magnetic resonance imaging system 100.

The operating console 101 may monitor a state of a static magnetic field, a state of a gradient magnetic field, a state of an RF signal, a state of an RF coil, a state of a table, a state of equipment for measuring biometric information of the subject (ob), a power supply state, a state of a heat exchanger, a state of a compressor, etc.

The magnet assembly 150 may be provided in the main body 105. The magnet assembly 150 includes a static magnetic field coil 151 for generating a static magnetic field in an inner space, the gradient coil 152 for generating a gradient in the static magnetic field to generate a gradient magnetic field, an RF transmitting coil 153, and an RF receiving coil 154.

The static magnetic field coil 151 may include coils wound around the bore 106. When an electric current is applied to the static magnetic field coil 151, a static magnetic field is generated inside the magnetic assembly 150, that is, at the bore 106. The direction of the static magnetic field is parallel to a driving axle of the magnetic assembly 150.

The gradient coil 152 forms a gradient in a static magnetic field, which is generated at the bore 106, and generates a gradient magnetic field.

As shown in FIG. 17, an axis parallel to a vertical direction from the head of the subject (ob) to the foot, that is, an axis parallel to the direction of the static magnetic field may be determined as a z-axis. An axis parallel to a horizontal direction of the subject (ob) may be determined as an x-axis. An axis parallel to the vertical direction in the space may be determined as a y-axis.

To obtain three-dimensional spatial information, a gradient magnetic field is applied in all of the x-axis, the y-axis, and the z-axis. Thus, the gradient coil 152 includes three pairs of gradient coils.

As shown in FIG. 18, a z-axis gradient coil 152z includes one pair of ring-shaped coils, and a y-axis gradient coil 152y is positioned above and below the subject (ob). An x-axis gradient coil 152x is positioned to the left and right of the subject (ob).

The RF transmitting coil 153 transmits an RF pulse, and an RF receiving coil 154 receives electromagnetic waves emitted from an excited atomic nucleus, that is, a magnetic resonance signal.

One RF coil may function as both of the RF transmitting coil 153 and the RF receiving coil 154. In this way, when the RF transmitting coil 153 and the RF receiving coil 154 are provided as one RF coil, the RF coil may alternately perform operations of the RF receiving coil and the RF transmitting coil.

The RF transmitting coil 153 may transmit an RF pulse to excite an atomic nucleus, and the RF receiving coil 154 may receive electromagnetic waves emitted from the atomic nucleus excited separately from the magnetic assembly 150, that is, a magnetic resonance signal.

When the subject (ob) is positioned in an inner space of the magnet assembly 150, a static magnetic field, a gradient magnetic field, and an RF pulse are applied by the static magnetic field coil 151, the gradient coil 152, and the RF transmitting coil 153 to excite the atomic nuclei constituting the subject (ob). A magnetic resonance signal is emitted from the atomic nuclei and then received by the RF receiving coil 154.

The data receiver 160 receives the magnetic resonance signal from the RF receiving coil 154 and delivers the received magnetic resonance signal to the main controller 120. Here, the data receiver 160 may correspond to the data collector 10 of FIG. 1.

The data receiver 160 may preprocess the received magnetic resonance data. In detail, the data receiver 160 may include a preamplifier for amplifying the magnetic resonance data received by the RF receiving coil 154, a phase detector for receiving the magnetic resonance signal from the preamplifier and performing phase detection, and an analog-to-digital (AD) converter for converting an analog signal acquired through the phase detection into a digital signal.

The data receiver 160 may store magnetic resonance data received from the RF receiving coil 154 or magnetic resonance data on which a predetermined process has been performed.

The main controller 120 controls the overall operation of the magnetic resonance imaging system 100 on the basis of information received from the operating console 101. In detail, the main controller 120 includes a static magnetic field controller 122 for controlling an intensity and direction of a static magnetic field generated by the static magnetic field coil 151 and a pulse sequence controller 123 for designing a pulse sequence and controlling the gradient coil 152 and the RF transmitting coil 153 according to the pulse sequence.

Here, the main controller 120 may correspond to the controller 20 of FIG. 1. That is, the main controller 120 may generate a protocol according to the respiration of the subject (ob), and the static magnetic field controller 122 and the pulse sequence controller 123 may control components according to a protocol.

The applicator 130 includes an RF applicator 130 and a gradient applicator 131. The RF applicator 130 applies an electrical current to the RF transmitting coil 153 based on the pulse sequence. The gradient applicator 130 applies an electrical current to the gradient coil 152 based on the pulse sequence.

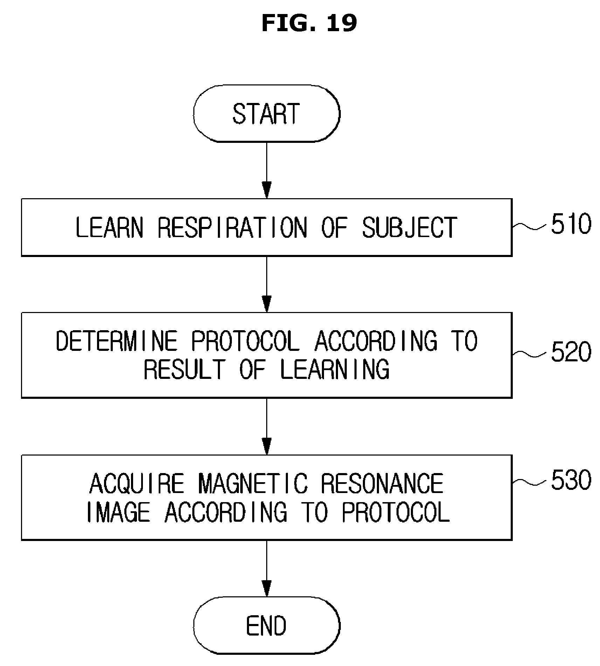

FIG. 19 is a flowchart showing a method of acquiring a magnetic resonance image in a magnetic resonance imaging system, according to an exemplary embodiment.

Referring to FIG. 19, the magnetic resonance imaging system 100 learns the respiration of the subject (ob) (operation 510). The main controller 120 may track the respiration of the subject (ob) and learn the respiration of the subject (ob) on the basis of the tracked respiration of the subject (ob). To track the respiration of the subject (ob), the main controller 120 may control components such that navigator data regarding a region corresponding to the thoracic diaphragm of the subject (ob) is acquired, create a navigator image using the navigator data acquired through the control, and track the respiration of the subject (ob) using a movement of the thoracic diaphragm acquired through the navigator image.

The main controller 120 may determine a respiratory cycle of the subject (ob), the timings of expiration and inspiration of the subject (ob), and the length of inspiration of the subject (ob). Such learning may be performed according to the above-described learning protocol 210.

The magnetic resonance imaging system 100 determines the protocol 200 according to a result of the learning (operation 520). The main controller 120 may set an image acquisition protocol 220 according to the result of the learning.

In detail, the main controller 120 may set the image acquisition protocol such that the image data of the subject (ob) is acquired during expiration. In addition, the main controller 120 may set the image acquisition protocol 220 such that the respiration of the subject (ob) is tracked when the image data of the subject (ob) is not acquired. That is, the main controller 120 may set the image acquisition protocol 220 such that the scanning period 221, the navigator period 222, and the idle period 223 are repeated on the basis of the result of the learning.

The magnetic resonance imaging system 100 acquires a magnetic resonance image according to the protocol 200 (operation 530). In detail, the static magnetic field controller 122 and the pulse sequence controller 123 of the main controller 120 output a control signal such that MR data is acquired according to the image acquisition protocol 220. The main controller 120 generates a magnetic resonance image of the subject (ob) using the acquired MR data.

In this way, it is possible to calculate an optimal image data acquisition timing and minimize an image data acquisition time by learning the respiration of the subject (ob) first and then setting the image acquisition protocol 220 according to the learned respiratory cycle of the subject (ob).

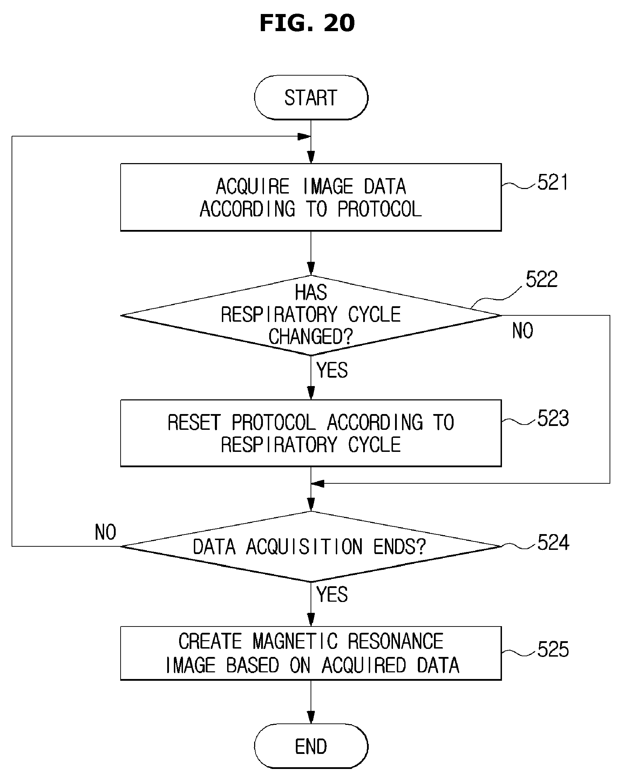

The main controller 120 may determine the change in respiration of the subject (ob) while acquiring the magnetic resonance image. When the respiration of the subject (ob) changes, the main controller 120 may reset the image acquisition protocol 220. In this way, it is possible to prevent deterioration in quality of the magnetic resonance image and minimize an acquisition time of the magnetic resonance image by dynamically changing the image acquisition protocol 220 according to the respiration of the subject (ob). This will be described below in detail with reference to FIG. 20.
FIG. 20 is a flowchart for describing in detail the method of acquiring a magnetic resonance image of FIG. 19.

Referring to FIG. 20, the magnetic resonance imaging system 100 acquires image data according to the protocol 200 (operation 521). In detail, the main controller 120 acquires the image data of the subject (ob) during inspiration according to the protocol 200. The acquisition of the image data is performed in units of slices. The image data is acquired until a k-space is entirely filled.

The magnetic resonance imaging system 100 determines whether the respiratory cycle has changed (operation 522). The main controller 120 may create a navigator image on the basis of navigator data acquired alternately with the image data, track the respiration of the subject (ob) using the created navigator image, and determine a change in the respiratory cycle or respiratory gradient of the subject (ob). A method of resetting the protocol 200 according to the change in the respiratory cycle will be described below. However, as described above, it is possible to reset the protocol 200 through a variety of information, such as the respiratory gradient, which may be derived from the respiration of the subject (ob).

For example, when the respiratory cycle of the subject (ob) changes (yes in operation 522), the magnetic resonance imaging system 100 resets the protocol 200 according to the changed respiratory cycle (operation 523). Although the respiratory gradient changes as described above, the magnetic resonance imaging system 100 may reset the protocol 200 according to the changed respiratory gradient and cannot reset the protocol using only the change in the respiratory cycle.

When the respiratory cycle changes (yes in operation 522), the magnetic resonance imaging system 100 cannot efficiently acquire the image data of the subject (ob) using the image acquisition protocol 220 that is set according to the respiratory information acquired from the learning. Accordingly, the main controller 120 resets the image acquisition protocol 220 on the basis of the changed respiratory cycle.

In detail, the main controller 120 may change the length of an acquisition time of the image data of the subject (ob) according to the changed respiratory cycle. That is, the main controller 120 changes the length of the scanning period 221 according to the changed respiratory cycle. The main controller 120 may change the image acquisition protocol 220 such that the main controller 120 decreases the acquisition time of the image data when the respiratory cycle shortens and increases the acquisition time of the image data when the respiratory cycle lengthens.

In detail, the main controller 120 may change the number of slices acquired during one cycle of the respiration according to the changed respiratory cycle. In detail, when the respiratory cycle shortens, the main controller 120 may reduce the number of slices acquired during one cycle of the respiration and thus decrease the acquisition time of the image data. On the other hand, when the respiratory cycle lengthens, the main controller 120 may increase the number of slices acquired during one cycle of the respiration and thus increase the acquisition time of the image data.

In detail, the main controller 120 may change the size of the slice acquisition window according to the changed respiratory cycle. In detail, when the respiratory cycle shortens, the main controller 120 may reduce the size of the slice acquisition window and thus decrease the acquisition time of the image data. On the other hand, when the respiratory cycle lengthens, the main controller 120 may increase the size of the slice acquisition window and thus increase the acquisition time of the image data.

The magnetic resonance imaging system 100 determines whether the data acquisition ends (operation 524).

When the data acquisition does not end (no in operation 524), the magnetic resonance imaging system 100 acquires the image data according to the protocol 200 (operation 521).

When the data acquisition ends (yes in operation 524), the magnetic resonance imaging system 100 creates a magnetic resonance image on the basis of the acquired image data (operation 525).

In this way, it is possible to enhance the quality of the magnetic resonance image and minimize the acquisition time of the magnetic resonance image by dynamically resetting the protocol 200 according to the respiratory cycle of the subject (ob).

In addition, the exemplary embodiments may also be implemented through computer-readable code and/or instructions on a medium, e.g., a computer-readable medium, to control at least one processing element to implement any above-described exemplary embodiments. The medium may correspond to any medium or media which may serve as a storage and/or perform transmission of the computer-readable code.

The computer-readable code may be recorded and/or transferred on a medium in a variety of ways, and examples of the medium include recording media, such as magnetic storage media (e.g., ROM, floppy disks, hard disks, etc.) and optical recording media (e.g., compact disc read only memories (CD-ROMs) or digital versatile discs (DVDs)), and transmission media such as Internet transmission media. Thus, the medium may have a structure suitable for storing or carrying a signal or information, such as a device carrying a bitstream according to one or more exemplary embodiments. The medium may also be on a distributed network, so that the computer-readable code is stored and/or transferred on the medium and executed in a distributed fashion. Furthermore, the processing element may include a processor or a computer processor, and the processing element may be distributed and/or included in a single device.

The foregoing exemplary embodiments are examples and are not to be construed as limiting. The present teaching can be readily applied to other types of apparatuses. Also, the description of the exemplary embodiments is intended to be illustrative, and not to limit the scope of the claims, and many alternatives, modifications, and variations will be apparent to those skilled in the art.

## Claims

1. A magnetic resonance imaging apparatus comprising:
a data acquisition unit configured to acquire image data of a subject according to a protocol;
a respiratory information acquisition unit configured to detect a respiration of the subject; and
a protocol control unit configured to, when a respiratory cycle of the subject changes, reset the protocol according to the changed respiratory cycle,
wherein the protocol includes a learning protocol for learning the respiration of the subject and determining an initial protocol.

2. The magnetic resonance imaging apparatus of claim 1, wherein the protocol control unit resets an acquisition time of the image data based on the changed respiratory cycle.

3. The magnetic resonance imaging apparatus of claim 1, wherein the protocol control unit changes a number of slices acquired during one cycle of the respiration based on the changed respiratory cycle.

4. The magnetic resonance imaging apparatus of claim 3, wherein the number of slices increases when the respiratory cycle lengthens, and decreases when the respiratory cycle shortens.

5. The magnetic resonance imaging apparatus of claim 4, wherein the protocol control unit changes the protocol such that a slice not acquired within the decreased number of slices is reacquired.

6. The magnetic resonance imaging apparatus of claim 1, wherein the protocol control unit changes a size of a slice acquisition window based on the changed respiratory cycle.

7. The magnetic resonance imaging apparatus of claim 6, wherein the size of the slice acquisition window increases when the respiratory cycle lengthens, and decreases when the respiratory cycle shortens.

8. The magnetic resonance imaging apparatus of claim 1, wherein the protocol control unit sets the protocol such that the detection of the respiration of the subject and the acquisition of the image data are alternately performed.

9. The magnetic resonance imaging apparatus of claim 1, wherein the protocol control unit changes the protocol such that the image data is acquired during an expiration of the changed respiratory cycle.

10. A method of acquiring a magnetic resonance image, the method comprising:
acquiring image data of a subject according to a protocol;
detecting a respiration of the subject; and
when a respiratory gradient of the subject changes, resetting the protocol according to the changed respiratory gradient,
wherein the resetting of the protocol comprises learning the respiration of the subject and determining an initial protocol according to the acquired respiratory gradient.

11. The method of claim 10, wherein the resetting of the protocol comprises resetting an acquisition time of the image data based on the changed respiratory gradient.

12. The method of claim 10, wherein the resetting of the protocol comprises changing a number of slices acquired during one cycle of the respiration based on the changed respiratory gradient.

13. The method of claim 12, wherein the number of slices increases when the respiratory gradient decreases, and decreases when the respiratory gradient increases.

14. The method of claim 13, further comprising acquiring a slice not acquired within the decreased number of slices.

15. The method of claim 10, wherein the resetting of the protocol comprises resetting a size of a slice acquisition window based on the changed respiratory gradient.
